(19) Europäisches Patentamt
European Patent Office
Office européen des brevets

(11) **EP 3 441 076 A1**

(12) **EUROPEAN PATENT APPLICATION**
published in accordance with Art. 153(4) EPC

(43) Date of publication:
**13.02.2019 Bulletin 2019/07**

(21) Application number: **16897546.4**

(22) Date of filing: **07.04.2016**

(51) Int Cl.:
*A61K 36/61* (2006.01)          *A61K 36/52* (2006.01)
*A61K 36/282* (2006.01)          *A61K 9/48* (2006.01)
*A61P 35/00* (2006.01)

(86) International application number:
**PCT/CN2016/078651**

(87) International publication number:
**WO 2017/173611 (12.10.2017 Gazette 2017/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **Beijing Lu De Kai Qi Co., Ltd.**
**Beijing 100000 (CN)**

• **Lu, Wei**
**Beijing 100000 (CN)**

(72) Inventor: **LU, Wei**
**Beijing 100000 (CN)**

(74) Representative: **Dr. Gassner & Partner mbB**
**Wetterkreuz 3**
**91058 Erlangen (DE)**

(54) **PHARMACEUTICAL PRODUCT FOR TREATING TUMOR AND COMBINED IMMUNE DEFECT, AND PREPARATION AND APPLICATION THEREOF**

(57) An anti-tumor pharmaceutical composition for combined immunization which comprises an extract of a walnut, an extract of a wormwood, and an extract of a clove in a weight ratio of 77:54:98. The composition can suppress microbial growth, kill microbes, enhance the immunity of the body, provide stronger anticancer activity, and prevent tumor recurrence. The composition is prepared using a supercritical fluid extraction process.

**EP 3 441 076 A1**

**Description**

**Technical Field**

The present invention relates to the field of medicine, and more particularly, to an anti-tumor pharmaceutical product for combined immunization, methods of preparation and use thereof.

**Background Art**

Malignant tumors, often called cancer, are common and frequently-occurring diseases that pose a serious threat to human health. At present, there are mainly three methods for treating malignant tumors including chemotherapy (abbreviated to chemo), surgical therapy and radiation therapy. The occurrence and development of malignant tumors are closely related to the immune function of bodies. Radiotherapy and chemotherapy will continuously weaken the immune function of bodies, affecting therapeutic effects. Chinese Patent No. CN1997361B discloses a group of anti-tumor pharmaceutical products, which, like most of chemotherapy, surgery and radiotherapy, can kill tumor cells, but they also kill normal cells at the same time, which leads to decline in patients' immunity, affects therapeutic effects, and also increases the probability of tumor recurrence.
Chinese Patent No. CN103301201B discloses a compound formula of Chinese herbal medicine that enhances immunity and assists in anti-tumor treatment. Although this compound formula of Chinese herbal medicine can improve immunity to a certain extent, it cannot repair patients' immunity that has been destroyed by previous treatment. Also, the compatibility between this Chinese herbal medicine and anti-tumor pharmaceutical products is poor.
How to repair and improve the immune function of the body while maximizing the killing of tumor cells so that the body can normalize or strengthen immune cells to kill residual tumor cells is the key to treating tumors.

**Summary of the Invention**

In view of the deficiencies in the prior art, the object of the present invention is to provide an anti-tumor pharmaceutical product for combined immunization which can suppress microbial growth, kill microbes, enhance the immunity of the body, provide relatively stronger anticancer biological activity, and prevent tumor recurrence.

In order to achieve the above object, the present invention provides the following technical solutions: An anti-tumor pharmaceutical product for combined immunization which is prepared from an extract of a walnut, an extract of a wormwood, and an extract of a clove, the extract of a walnut, the extract of a wormwood, and the extract of a clove are in a weight ratio of 77:54:98.

Preferably, the walnut is black walnut, the wormwood is *Artemisia absinthium,* and the clove is clove bud. Walnut is *Julans regia L.* belonging to *Juglandaceae.* The walnut extract according to the present invention is prepared by extracting the endodermis of Black Walnut (*Juglans nigra L*), which contains juglone, flavones, tannin, polysaccharide, gallic acid, volatile oil, mucilaginous gum, albumin, mineral, cellulose, linoleic acid, glyceride, linolenic acid, glyceryl oleate, riboflavin, carotene, gemin D, casurin, and extract from *Rosa davurica Pall..* It has been found that different ratios of the active ingredients can achieve the object of the present invention, and the extract prepared by the preparation method according to the present invention is the most effective.

Wormwood, also known as folium artemisiae argyi, felon herb, Chinese mugwort or Jia Ai, is a dry leave of *Blumea aromatica Levi.et Vant.* belonging to *Compositae.* It is pungent, bitter and warm, has slight toxicity and can dispel coldness to relieve pain and warming meridian to stop bleeding. The wormwood extract according to the present invention is preferably prepared by extracting the whole herb of *Artemisia absinthium* of North America (wormwood or *Artemisia absinthium*). The main component of this extract is volatile oil, and the oil contains terpinenol-4, β-caryophyllene, artemisia alcohol, linalool, camphorae, borneol, cineol (eucalyptol), trans-carveol, α-terpinenol, etc., and polysaccharides, tannic acid, thujone, phellandrene, β-sitosterol, 5,7-dihydroxy-6,3',4'-trimethyl flavone. A study has found that the difference in ratios of active ingredients can achieve the object of the present invention, and the wormwood extract prepared by the preparation method according to the present invention is most effective.

Clove, also known as male clove or clove bud, is the dried flower bud of *Eugenia caryophyllata Thumb.* belonging to *Myrtaceae.* It has warm nature with pungent flavor and can warming middle energizer to descend adverse-rising qi, tonify kidney and support yang. The clove extract according to the present invention is preferably prepared by extracting dried and unopened flower buds of *Eugenia caryphyllata* mainly produced in the tropical and subtropical regions of Australia and the America. The main ingredients of this extract is volatile oil, and the oil mainly contains eugenol, caryophyllene, and acetyleugenol, and also contains 2-heptanone (methyl-n-amyl-ketone), salicylic acid-formaldehyde, α-caryophyllene, ylangene, chavicol, syringic acid, ethyl syringate, oleanolic acid, kaempferol, rhamnetin, benzopyrones compound such as eugenin, eugenitin, isoeugenitin, isoeugenitol, sitosterol, tannin, vanillin, green leaf waxiness, organic acids, flavones, proteins, etc. It has been found that difference in the ratios of active ingredients can achieve the object of the present invention, and the clove extract prepared by the preparation method according to the present invention is most effective.

It has been found that the weight ratio of an extract from walnuts, an extract from wormwoods, and an extract from cloves in the anti-tumor pharmaceutical product for combined immunization according to the present invention is 77:54:98 and their combination has the following synergistic effects:

(1) relieving and improving symptoms in patients with tumor, and increasing their quality of life, such as relieving pain, increasing appetite, analgesia and replenishing blood;

(2) bacteriostasis, sterilization, antivirus, having strong anti-oxidation and hydroxyl radical scavenging ability, and enhancing the ability of patients with tumor to fight against other new diseases;

(3) directly stimulating the division, differentiation and maturation of bone marrow stem cells, maintaining a normal number of white blood cells and platelets in the body, resisting inflammation and hemostasis, stimulating cellular immune regulatory factors, enhancing cellular immune activity, regulating internal environment of the body and restoring normal physiological balance to ensure that the treatment of tumor can be carried out normally;

(4) having strong affinity for tumor cells, being capable of modifying tumor cells, changing the characteristics of malignant cell membrane, having an extremely strong killing effect on tumor cells, having relatively strong anticancer activity, being capable of interfering with the division cycle of tumor cells, stagnating the growth of tumor cells in the prophase, reducing the tumor size or even disappearing it;

(5) repairing the damaged immunity due to chemotherapy and the like, improving immune hypofunction or inhibiting immune hyperfunction, regulating the internal environment of the body, maintaining the immune function balance, and preventing the recurrence of tumors.

Preferably, the formulation of the pharmaceutical product is a capsule.

Administration in the form of capsule is advantageous for ensuring that the anti-tumor pharmaceutical product for combined immunization according to the present invention can be administered accurately and conveniently, which is advantageous for control.

It has been found that when the walnut extract, the wormwood extract, and the clove extract are combined with paclitaxel, the anti-tumor ability can be further improved.

Another object of the present invention is to provide a method of preparing the anti-tumor pharmaceutical product for combined immunization, the method comprises the preparation of a walnut extract capsule, the preparation of a wormwood extract capsule, and the preparation of a clove extract capsule; the preparation of a walnut extract capsule, the preparation of a wormwood extract capsule, and the preparation of a clove extract capsule include:

S1, screening and washing raw materials, and softening the clean raw materials by cold dipping under vacuum;

S2, subjecting the raw material softened in the step S1 to supercritical fluid extraction and separating to obtain a liquid extract and a residue;

S3, pulverizing and grinding the residue obtained in step S2;

S4, spray drying the liquid extract obtained in step S2 and adding the pulverized and ground residue obtained in step S3 thereto during the spray drying to obtain a dried extract of raw materials; and

S5, filling a formulated amount of the extract of raw materials obtained in the step S4 into a capsule shell to obtain a capsule.

The softening method by cold dipping under vacuum is applied in the present invention, which rapidly introduces water by the action of negative pressure so as to accelerate the softening of medicinal materials, ensures softening of medicinal materials with water at normal temperature, shortens the infiltration time, reduces loss and damage of active ingredients and decreases mildewing of medicinal materials.

The supercritical fluid extraction technology is applied in the present invention for extracting medicinal plants, in which the fluid is carbon dioxide and the extraction temperature is 30-40°C. This technology can avoid the volatilization of volatile oil and other ingredients during the extraction process, and can maximally maintain the effective bioactive ingredients of medicinal plants. At the same time, there is no solvent residue since the organic solvent is not applicable in the process, which ensures the natural biological activity of the raw materials, environmental protection, safety and no side effects.

The spray drying is applied in the present invention for quickly drying the liquid extract of raw materials of the invention, and the obtained product has fine and uniform particle size, good fluidity, increased specific surface area, favorable dissolution in the body, and favorable absorption by the human body. The liquid extract of raw materials is spray-dried to obtain fine particle material. Since the particle material contains substances with larger viscosity such as sugars, proteins or starches, the residue of the raw materials is added during spray drying of the liquid extract of raw materials. The residue of the raw materials can adsorb the spray-dried liquid extract of raw materials, and the spray-dried liquid extract of raw materials has strong binding force with the residue of the raw materials, which can prevent the occurrence of wall sticking. At the same time, the raw material medicine is fully utilized, and no other extra auxiliary materials are added, which are conducive to maintain its natural activity.

The capsule prepared by the above preparation has safety and no side effects. The extraction process does not destroy the original active components of the plants, and the active components can be fully made into an extract having a large specific surface area, which is beneficial to dissolution in the body and absorption by the human body. At the same time, it is possible to avoid the wall-sticking phenomenon or the like which occurs during the production process by using this method.

Preferably, it has been found that in S 1, the cold dipping under vacuum is performed at the temperature of 10-40°C and at the pressure of -0.1 to -0.09 MPa;

in S2, a fluid for supercritical fluid extraction is carbon dioxide, and the extraction temperature is 30-40°C;

in S4, the temperature of spray drying is 40-80°C, the drying time is 3-60 s, and the extract of raw materials has a particle size of 10-60 $\mu$m; and

in S5, the capsule shell is a capsule shell of natural plants.

Another object of the present invention is to provide a method of preparing the anti-tumor pharmaceutical product for combined immunization, the method comprises preparation of a black walnut extract capsule, preparation of a wormwood extract capsule, and preparation of a clove extract capsule;

the preparation of a black walnut extract capsule comprises:

the method comprises preparation of a walnut extract capsule, preparation of a wormwood extract capsule, and preparation of a clove extract capsule;

the preparation of the walnut extract capsule comprises:

S1, screening and washing a walnut, and softening clean endodermis of the walnut by cold dipping under vacuum; the cold dipping under vacuum is performed at the temperature of 10-40°C and at the pressure of -0.1 to -0.09 MPa;

S2, subjecting the walnut softened in the step S1 to supercritical fluid extraction and separating to obtain a liquid extract of a walnut and a walnut residue; a fluid for supercritical fluid extraction is carbon dioxide, and the extraction temperature is 30-40°C;

S3, pulverizing and grinding the walnut residue obtained in step S2;

S4, spray drying the liquid extract of a walnut obtained in step S2 and adding the pulverized and ground walnut residue obtained in step S3 during the spray drying to obtain a dried extract of a walnut; in which the temperature of spray drying is 40-80°C, the drying time is 3-60 s, and the extract of a walnut has a particle size of 10-60 $\mu$m; and

S5, filling 77 parts of the extract of a walnut obtained in the step S4 into a natural plant capsule shell to obtain a walnut extract capsule;

the preparation of the wormwood extract capsule comprises:

S1, screening and washing a wormwood, and softening clean whole herb of the wormwood by cold dipping under vacuum; in which the cold dipping under vacuum is performed at the temperature of 10-40°C and at the pressure of -0.1 to -0.09 MPa;

S2, subjecting the wormwood softened in the step S1 to supercritical fluid extraction and separating to obtain a liquid extract of a wormwood and an wormwood residue; the fluid for supercritical fluid extraction is carbon dioxide, and the extraction temperature is 30-40°C;

S3, pulverizing and grinding the wormwood residue obtained in step S2;

S4, spray drying the liquid extract of a wormwood obtained in step S2 and adding the pulverized and ground wormwood residue obtained in step S3 thereto during the spray drying to obtain a dried extract of a wormwood; the temperature of spray drying is 40-80°C, the drying time is 3-60 s, and the extract of a wormwood has a particle size of 10-60 $\mu$m; and

S5, filling 54 parts of the extract of a wormwood obtained in the step S4 into a natural plant capsule shell to obtain a wormwood extract capsule;

the preparation of the clove extract capsule comprises:

S1, screening and washing a clove, and softening the clean and unopened flower buds of the clove by cold dipping under vacuum; in which the cold dipping under vacuum is performed at the temperature of 10-40°C and at the pressure of -0.1 to -0.09 MPa;

S2, subjecting the clove softened in the step S1 to supercritical fluid extraction and separating to obtain a fluid extract of a clove and a clove residue; a fluid for supercritical fluid extraction is carbon dioxide, and the extraction temperature is 30-40°C;

S3, pulverizing and grinding the clove residue obtained in step S2;

S4, spray drying the extract of a clove obtained in step S2 and adding the pulverized and ground clove residue obtained in step S3 thereto during the spray drying to obtain a dried extract of a clove; the temperature of spray drying is 40-80°C, the drying time is 3-60 s, and the extract of a clove has a particle size of 10-60 μm; and

S5, filling 98 parts of the extract of a clove obtained in the step S4 into a natural plant capsule shell to obtain a clove extract capsule; and

the walnut is black walnut, the wormwood is *Artemisia absinthium,* and the clove is clove bud.

In the anti-tumor pharmaceutical product for combined immunization according to the present invention, the black walnut extract capsule, the Artemisia absinthium extract capsule, and the clove bud extract capsule are separately prepared. In an aspect, the mutual interference of the three plants can be avoided during the treatment, which is beneficial to ensure the quality of the product. In another aspect, at the time of treatment, the administration of the pharmacuical product can be varied according to the needs, which is suitable for different types of patients.

Another object of the present invention is to provide use of an anti-tumor pharmaceutical product for combined immunization in the manufacture of a pharmaceutical product for preventing or treating cancers, and the cancers comprises intestinal cancer, mastocarcinoma, gastric cancer, liver cancer, prostate cancer, breast cancer, lung cancer, cervical cancer, ovarian cancer, kidney cancer, lymphoma, leukemia, skin cancer, melanoma, stromal cell tumor, myeloma or astrocytoma.

Use of an anti-tumor pharmaceutical product for combined immunization prepared by the preparation method in the manufacture of pharmaceutical products for preventing or treating cancers, and the cancers comprise intestinal cancer, mastocarcinoma, gastric cancer, liver cancer, prostate cancer, breast cancer, lung cancer, cervical cancer, ovarian cancer, kidney cancer, lymphoma, leukemia, skin cancer, melanoma, stromal cell tumor, myeloma or astrocytoma of the brain.

The following beneficial effects are obtained by using the above technical solutions:

1. The anti-tumor pharmaceutical product for combined immunization according to the present invention has the following synergistic effects: in an aspect, bacteriostasis, sterilization, killing tumor cells and having strong anticancer activity; in another aspect, enhancing the immunity of the body, regulating the internal environment of the body, and restoring the normal balance of the body. This can ensure that the immunity of the body can be enhanced and the destruction of immune system of the body can be avoided during the treatment of cancer, which is beneficial to guarantee anti-cancer effects and further prevent tumor recurrence.

2. In the preparation process of the anti-tumor pharmaceutical product for combined immunization according to the present invention, the effective bioactive components of the medicinal plants can be extracted to the greatest extent without using organic solvents and destructive conditions, and the components are safe and have no side effects. Meanwhile, the effective bioactive components can also be made into the particles with a large specific surface area, which is beneficial to the absorption by human body.

3. In the preparation process of the anti-tumor pharmaceutical product for combined immunization according to the present invention, the residue of the raw materials is added during the spray drying of the liquid extract of raw materials, which contributes to solve the wall-sticking problem of the extract of raw materials during the production process together with conventional anti-sticking operation, and to fully utilize the raw material medicine to maintain its natural activity.

4. The anti-tumor pharmaceutical product for combined immunization according to the present invention is in the form of a capsule, and thus the administration is precise and convenient, which is advantageous for preservation and stability.

**Examples**

In the examples, the walnut is selected from Black Walnut (also called Juglans nigra L). The wormwood is selected from Artemisia absinthium (wormwood or Artemisia absinthium). The clove is selected from Eugenia caryphyllata mainly produced in the tropical and subtropical regions of Australia and the America.

The endodermis of walnut was screened and washed, and the clean endodermis of walnut was softened by cold dipping under vacuum at the temperature of 10-40°C and the pressure of -0.1 to -0.09 MPa. After softening, the supercritical fluid extraction was carried out at 30-40°C using a carbon dioxide fluid, and a liquid extract of a walnut and a walnut residue were obtained by separating. The walnut residue was pulverized, ground and passed through a 200-300 mesh sieve. The liquid extract of a walnut was sprayed onto the sieved walnut residue by spray drying at a temperature of 40 to 80°C for a drying time of 3 to 60 s to obtain an extract of a walnut having a particle size of 10-60 $\mu$m. The extract of a walnut was filled into a natural plant capsule shell by a conventional capsule filling method to obtain a walnut extract capsule.

The whole herb of wormwood was screened and washed, and the clean whole herb of wormwood was softened by cold dipping under vacuum at the temperature of 10-40°C and the pressure of -0.1 to -0.09 MPa. After softening, the supercritical fluid extraction was carried out at 30-40°C using a carbon dioxide fluid, and a liquid extract of wormwoods and a wormwood residue were obtained by separating. The wormwood residue was pulverized, ground and passed through a 200-300 mesh sieve. The liquid extract of wormwoods was sprayed onto the sieved wormwood residue by spray drying at a temperature of 40 to 80°C for a drying time of 3 to 60 s to obtain a wormwood extract having a particle size of 10-60 $\mu$m. The wormwood extract was filled into a natural plant capsule shell by a conventional capsule filling method to obtain a wormwood extract capsule.

The dry and unopened flower buds of clove (Eugenia caryphyllata)was screened and washed, and the clean unopened flower buds of clove was softened by cold dipping under vacuum at the temperature of 10-40°C and the pressure of -0.1 to -0.09 MPa. After softening, the supercritical fluid extraction was carried out at 30-40°C using a carbon dioxide fluid, and a liquid extract of a clove and a clove residue were obtained by separating. The clove residue was pulverized, ground and passed through a 200-300 mesh sieve. The liquid extract of the clove was sprayed onto the sieved clove residue by spray drying at a temperature of 40 to 80°C for a drying time of 3 to 60 s to obtain a clove extract having a particle size of 10-60 $\mu$m. The clove extract was filled into a natural plant capsule shell by a conventional capsule filling method to obtain a clove extract capsule.

The walnut extract capsule, the wormwood extract capsule, and the clove extract capsule are simultaneously administered in a certain ratio when using anti-tumor pharmaceutical product for combined immunization according to the present invention.

Table 1 The anti-tumor pharmaceutical product for combined immunization according to the present invention

| Example<br>Extract capsule | | Example 1 | Example 2 | Example 3 |
|---|---|---|---|---|
| walnut extract capsule | Mass of walnut extract (mg) | 385 | 289 | 192 |
| | Type No. of natural plant capsule shell | 0# | 1# | 3# |
| wormwood extract capsule | Mass of wormwood extract (mg) | 270 | 202 | 135 |
| | Type No. of natural plant capsule shell | 0# | 1# | 3# |
| clove extract capsule | Mass of clove extract (mg) | 490 | 367 | 245 |
| | Type No. of natural plant capsule shell | 0# | 1# | 3# |

Comparative example 1. Toxicology assay

1. Test materials: The walnut extract, the wormwood extract, and the clove bud extract were all prepared in the same manner as in Example 1. The walnut extract, the wormwood extract, and the clove bud extract were respectively subjected to a water bath at 70°C for 1 hour, soaked for 12 hours, and concentrated for testing.

2. Test animals:

The healthy Kunming mice provided by the Experimental Animal Center of the National Institute for the Control of Pharmaceutical and Biological Products were selected.

3. Test for micronucleated polychromatic erythrocytes in bone marrow of mice

The test was carried out via oral gavage and the administration was performed twice at intervals of 24 hrs. 50 mice weighing 25-30 g were randomly divided into 5 groups according to body weight, 10 in each group, half male and half female. The cyclophosphamide (cp) at a dose of 50 mg/kg•bw was used as a positive control, and the distilled water was used as a negative control. The doses of the anti-tumor pharmaceutical product for combined immunization according to the present invention were 1.25, 2.50, and 5.00 g/kg•bw, respectively, and the desired concentrations were adjusted with distilled water. The animals were sacrificed by cervical dislocation six hours after the last administration of the anti-tumor pharmaceutical product for combined immunization according to the present invention. The bone marrow of sternum was acquired and diluted with calf serum, smeared, fixed with methanol, and stained with Giemsa. Under the light microscope, 1000 polychromatic erythrocytes (PRC) were counted per animal, and the incidence of micronucleus was calculated by the permillage of micronucleus-containing PRC and statistically processed. The statistical results for the effects of the anti-tumor pharmaceutical product for combined immunization according to the present invention on the incidence of bone marrow micronucleus in mice are shown in Table 2.

Table 2 Statistical results of test for micronucleated polychromatic erythrocytes in bone marrow of mice

| Gender | Dose (g/kg•bw) | Number of animals (in Zhi) | Number of cells as examined (in Ge) | Number of micronucleus (in Ge) | Micronucleus rate (‰) |
|---|---|---|---|---|---|
| Male | 0.00 | 5 | 5000 | 9 | 1.8 |
| | 1.25 | 5 | 5000 | 7 | 1.4 |
| | 2.50 | 5 | 5000 | 8 | 1.6 |
| | 5.00 | 5 | 5000 | 9 | 1.8 |
| | 50 mg/kg•bw (cp) | 5 | 5000 | 164 | 32.8* |
| Female | 0.00 | 5 | 5000 | 8 | 1.6 |
| | 1.25 | 5 | 5000 | 8 | 1.6 |
| | 2.50 | 5 | 5000 | 9 | 1.8 |
| | 5.00 | 5 | 5000 | 10 | 2.0 |
| | 50 mg/kg•bw (cp) | 5 | 5000 | 144 | 28.8* |
| *Extremely significant difference from the negative control group (P<0.01). | | | | | |

It can be seen from table 2 that the micronucleus rate of each dose group of the anti-tumor pharmaceutical product for combined immunization according to the present invention is small, and there is no significant difference between the respective dose groups and the negative control group, while the difference between the cyclophosphamide group and the negative control group is extremely significant (P<0.01). The anti-tumor pharmaceutical product for combined immunization according to the present invention does not affect the rate of micronucleated polychromatic erythrocytes in bone marrow of mice.

4. Sperm abnormality assay in mice

Fifty sexually mature male mice weighing 30-35 g were randomly divided into 5 groups. The cyclophosphamide at a dose of 40 mg/kg•bw was used as a positive control, and the distilled water was used as a negative control. The doses of the anti-tumor pharmaceutical product for combined immunization according to the present invention were 1.25, 2.50, and 5.00 g/kg•bw, and the desired concentration were adjusted with distilled water. The mice were administered via oral gavage once a day for 5 days, and the animals were sacrificed 30 days after the last gavage. The epididymis was acquired and sectioned and stained with eosin. Five animals were counted in each group and 1000 intact sperm were counted per animal. The incidence of sperm abnormality (in percentage) was calculated and statistically processed. The statistical results of the effects of the anti-tumor pharmaceutical product for combined immunization according to the present invention on the incidence of sperm abnormality in mice are shown in Table 3.

Table 3 Statistical results of sperm abnormality assay in mice

| Dose (g/ kg•bw) | Number of animals (in Zhi) | Number of sperms as examined (in Ge) | Number of abnormal sperms (in Ge) | Rate of sperm abnormality (%) |
|---|---|---|---|---|
| 0.00 | 5 | 5000 | 100 | 2.00 |
| 1.25 | 5 | 5000 | 94 | 1.88 |
| 2.50 | 5 | 5000 | 99 | 1.98 |
| 5.00 | 5 | 5000 | 101 | 2.02 |
| 40 mg/ kg•bw (cp) | 5 | 5000 | 517 | 10.34* |
| * Extremely significant difference from the negative control group (P<0.01). | | | | |

It can be seen from table 3 that the anti-tumor pharmaceutical product for combined immunization according to the present invention does not significantly affect the incidence of sperm abnormality in the mice, and there is no significant difference between the respective dose groups and the negative control group, while the difference between the cyclophosphamide group and the negative control group is extremely significant (P<0.01). Therefore, the anti-tumor pharmaceutical product for combined immunization according to the present invention does not affect the sperm abnormality in the mice.

The anti-tumor pharmaceutical products for combined immunization according to the present invention as prepared in Examples 2-3 were also subjected to the same toxicological assay, and the results were consistent with that of the anti-tumor pharmaceutical product for combined immunization according to the present invention as prepared in Example 1.

Comparative example 2. Immunomodulatory assay

1. Test materials: The walnut extract capsule, the wormwood extract capsule, and the clove extract capsule were all prepared in the same manner as in Example 1. The recommended amount for oral administration in human is 2.1 g/day, which is calculated based on 60 kg body weight per person (one capsule for each variety), and equivalent to 0.035 g/kg•bw. The equivalent dose in the mouse is equal to 10 times the recommended amount for the human body, that is, 0.35 g/kg•bw (medium dose) of shaped product per day. A group of high dose and a group of low dose were set, respectively: 1.05 g/kg•bw (high dose) of product and 0.035 g/kg•bw of product (low dose). The samples were formulated in sterilized water and sterilized water was used as control group. After continuous gavage for 30 days, various immune indicators were measured. Since the walnut extract capsule, the wormwood extract capsule, and the clove extract capsule are all insoluble in water, these capsules were soaked with 10 times the recommended amount of hot water (70-80°C) for 3 times (one hour/time) and then were concentrated by evaporation on a rotary evaporator (50-60°C) to a concentration of 30 times the recommended dose, and then diluted proportionally for gavage.

2. Test animals:

Seventy-five healthy male Kunming mice weighing 18-22 g provided by the Experimental Animal Center of the National Institute for the Control of Pharmaceutical and Biological Products were selected and divided into 4 groups with 15 per group for the dose groups and 30 for the control group to carry out delayed type hypersensitivity and antibody-forming cell assays.

Seventy-five healthy male Kunming mice weighing 18-22 g provided by the Experimental Animal Center of the National Institute for the Control of Pharmaceutical and Biological Products were selected and divided into 4 groups with 15 per group for the dose groups and 30 for the control group to carry out carbon clearance assay.

3. Delayed type hypersensitivity assay (DTH) (toe incrassation)

The mice were intraperitoneally injected with 2% (v/v, formulated with physiological saline) of SRBC (0.2 mL/mouse). Four days after sensitization, the thickness of the left and right footpads was measured, the same position was measured three times and the results were averaged. Then, 20% (v/v, formulated with physiological saline) of SRBC (20 µL/mouse) was injected subcutaneously at the measurement site, and the thickness of the left and right footpads were measured 24 hours after the injection, the same position was measured three times and the results were averaged. The degree of DTH was expressed by the difference in the thickness of the footpads before and after the injection (swelling degree of the footpads). The statistical results of the effects of the anti-tumor pharmaceutical product for combined immunization according to the present invention on delayed type hypersensitivity (DTH) in mice are shown in Table 4.

Table 4. Statistical results of delayed type hypersensitivity assay (DTH)

| Groups | Number of animals | Swelling degree of the footpad (mm) |
| --- | --- | --- |
| Control | 30 | 0.523 ± 0.109 |
| Low dose | 15 | 0.683 ± 0.106 |
| Medium dose | 15 | 0.737 ± 0.127 |
| High dose | 14 | 0.703 ± 0.111 |

The mice were orally administered with different doses of the anti-tumor pharmaceutical product for combined immunization according to the present invention for 30 days, and the results were statistically processed. As seen from Table 4, as for the swelling degree of the footpad, there was an extremely significant difference between the low, medium and high dose groups and the control group ($P<0.01$). In other words, the anti-tumor pharmaceutical product for combined immunization according to the present invention can significantly enhance the delayed type hypersensitivity in mice.

4. Antibody-forming cell assay (modified Jerne plaque assay)

Mice were immunized by intraperitoneal injection of 0.2 mL of 2% (v/v, formulated with physiological saline) of SRBC (0.2 mL per mouse). After 5 days, the mice were sacrificed, and their spleens were removed, shredded and prepared as a cell suspension using Hank's solution. the cell suspension was washed, centrifuged twice, and suspended in 5 mL of Hank's solution. After heating and dissolving the surface medium (agarose), it was mixed with an equal amount of double Hank's solution and dispensed into small tubes with 0.5 ml per tube. 10% (v/v, prepared with SA solution) of SRBC 50 $\mu$L and 20 $\mu$L spleen cell suspension were added to the tubes. After quickly mixing, the mixture was poured onto a slide coated with agarose. After the agar solidified, the slide was placed horizontally on the slide holder and incubated in a carbon dioxide incubator for 1.5 hr. The complement diluted with SA buffer (1:10) was added to the groove of the slide holder. After incubation for 1.5 hr, the number of hemolysis plaques was counted. The statistical results of the effects of the anti-tumor pharmaceutical product for combined immunization according to the present invention on the number of antibody-forming cells are shown in Table 5.

Table 5 Statistical results of antibody-forming cell assay

| Groups | Number of animals (in Zhi) | Number of hemolysis plaques ($\times 10^3$/a whole spleen) |
| --- | --- | --- |
| Control | 22 | 111.8 ± 60.8 |
| Low dose | 11 | 102.2 ± 45.6 |
| Medium dose | 13 | 131.6 ± 62.3 |
| High dose | 12 | 129.6 ± 101.3 |

4. Antibody-forming cell assay (modified Jerne plaque assay)

Mice were immunized by intraperitoneal injection of 0.2 mL of 2% (v/v, formulated with physiological saline) of SRBC (0.2 mL per mouse). After 5 days, the mice were sacrificed, and the spleen was removed shredded gently, and was prepared as a cell suspension using Hank's solution, which the cells were washed, centrifuged twice, and suspended in 5 mL of Hank's solution. After heating and dissolving the surface medium (agarose), it was mixed with an equal amount of double Hank's solution and dispensed into small tubes with 0.5 mL per tube. 10% (v/v, formulated with SA solution) of SRBC 50 $\mu$L and 20 $\mu$L spleen cell suspension were added to the tubes. After quickly mixing, the mixture was poured onto a slide coated with agarose. After the agar solidified, the slide was placed horizontally on the slide holder and incubated in a carbon dioxide incubator for 1.5 hr. The complement diluted with SA buffer (1:10) was added to the groove of the slide holder. After incubation for 1.5 hr, the number of hemolysis plaques was counted. The statistical results of the effects of the anti-tumor pharmaceutical product for combined immunization according to the present invention on the number of antibody-forming cells are shown in Table 5.

The mice were orally administered with different doses of the anti-tumor pharmaceutical product for combined immunization according to the present invention for 30 days, and the results were statistically processed. As seen from Table 5, as for the swelling degree of the footpad, there was no extremely significant difference between the low, medium and high dose groups and the control group (P>0.05). In other words, the anti-tumor pharmaceutical product for combined immunization according to the present invention does not affect the number of antibody-forming cells in mice.

5. Carbon clearance assay in mice

The tail vein of the mice was injected with Indian ink diluted with physiological saline (1:4) at a dose of 0.1 mL/10 g body weight, and timing was started immediately after the injection. 20 $\mu$L of blood was taken from the angular vein 2 and 10 minutes after the injection, added into 2 mL of 0.1% $Na_2CO_3$ solution, and shaken well. The 0.1% $Na_2CO_3$ solution was used as a blank control, and the optical density value (OD) was measured at a wavelength of 600 nm using a 721 spectrophotometer. The mice were sacrificed, the liver and spleen were removed and weighed (body weight is expressed in m, liver weight is expressed in $m_1$, spleen weight is expressed in $m_2$), and the phagocytic index a was calculated as follows:

$$K = \frac{\lg OD_1 - \lg OD_2}{t_2 - t_1} \qquad (\text{Formula } 1-1)$$

$$a = \frac{m}{m_2 + m_1} \times \sqrt[3]{k} \qquad (\text{Formula } 1-2)$$

The statistical results of the effects of the anti-tumor pharmaceutical product for combined immunization according to the present invention on the monocyte-macrophage phagocytosis in mice are shown in Table 6.

Table 6 Statistical results of carbon clearance assay in mice

| Groups | Number of animals (in Zhi) | Phagocytic index |
|---|---|---|
| Control | 26 | 5.97 $\pm$ 0.60 |
| Low dose | 15 | 6.33 $\pm$ 0.40 |
| Medium dose | 14 | 5.88 $\pm$ 0.58 |
| High dose | 14 | 5.87 $\pm$ 0.63 |

The mice were orally administered with different doses of the anti-tumor pharmaceutical product for combined immunization according to the present invention for 30 days, and the results were statistically processed. As seen from Table 6, as for the swelling degree of the footpads, there was no extremely significant difference between the low, medium and high dose groups and the control group (P>0.05). In other words, the anti-tumor pharmaceutical product for combined immunization according to the present invention does not affect the monocyte-macrophage carbon clearance in mice.

The mice were orally administered with different doses of the anti-tumor pharmaceutical product for combined immunization according to the present invention for 30 days. As seen from Tables 4-6, the anti-tumor pharmaceutical product for combined immunization according to the present invention can significantly enhance the delayed type hypersensitivity in mice, but does not affect the number of antibody-forming cells and the monocyte-macrophage carbon clearance in mice. Therefore, the anti-tumor pharmaceutical product for combined immunization according to the present invention has an effect of enhancing cellular immune function.

The anti-tumor pharmaceutical products for combined immunization according to the present invention as prepared in Examples 2-3 were also subjected to the same immunomodulatory assay, and the results were consistent with that of the anti-tumor pharmaceutical product for combined immunization according to the present invention as prepared in Example 1.

Comparative example 3. Hygienic inspection of preparations

With reference to GB4789-94 and GB4789.15-94, the anti-tumor pharmaceutical product for combined immunization according to the present invention as prepared in Example 1 was tested for coliform bacteria, total number of colonies, pathogenic bacteria, mold, and yeast count. A set of samples just produced and a set of samples placed at 25°C/60% RH for 3 years were tested separately, and both sets of samples were samples from the same production batch. Ten samples were taken in parallel for each set of samples. The hygienic inspection results are shown in Table 7.

Table 7 Statistical results of hygienic inspection of preparations

| Source of sample | walnut extract capsule | | wormwood extract capsule | | clove extract capsule | |
|---|---|---|---|---|---|---|
| | 0 day | 3 years | 0 day | 3 years | 0 day | 3 years |
| Coliform bacteria (MPN/100 g) | <30 | <30 | <30 | <30 | <30 | <30 |
| Total number of colonies (CFU/g) | <10 | <10 | <10 | <10 | <10 | <10 |
| Staphylococcus aureus | Not detected | Not detected | Not detected | Not detected | Not detected | Not detected |
| Streptococcus hemolyticus | Not detected | Not detected | Not detected | Not detected | Not detected | Not detected |
| Shigella | Not detected | Not detected | Not detected | Not detected | Not detected | Not detected |
| Salmonella | Not detected | Not detected | Not detected | Not detected | Not detected | Not detected |
| Mold (CFU/g) | Not detected | Not detected | Not detected | Not detected | Not detected | Not detected |
| Yeast (CFU/g) | Not detected | Not detected | Not detected | Not detected | Not detected | Not detected |

As can be seen from Table 7, the anti-tumor pharmaceutical products for combined immunization just produced and the anti-tumor pharmaceutical products for combined immunization placed at 25°C/60% RH for 3 years passed hygienic inspection.

The anti-tumor pharmaceutical products for combined immunization according to the present invention as prepared in Examples 2-3 were also subjected to the same hygienic inspection, and the results were consistent with that of the anti-tumor pharmaceutical product for combined immunization according to the present invention as prepared in Example 1.

Comparative example 4. Clinical trials

Clinical trials were performed in medium and advanced cancer patient cases. The criteria for inclusion were as follows:

(1) The diagnosis was confirmed by pathology, cytology or combining with clinical imaging.
(2) KPS score was $\geq$ 50 points and the patients were expected to survive for 6 months.
(3) The radiotherapy or chemotherapy or other treatments was suspended for more than one month.

The diagnosis was performed by combining the following means: routine examination of blood, urine and feces, sputum examination, exfoliated cell examination by direct sputum smear and staining, chest and ascites examination, stomach and duodenal fluid examination, biochemical examination, chemical or immunological methods for tumor marker, imaging examination (including ordinary x-ray examination, B-type ultrasonic examination, radionuclide examination, CT, MRI, PET, etc.), endoscopy, and pathological examination (including exfoliated cell examination, tissue imprints with staining, pathological section examination).

A total of 200 medium and advanced cancer patients were involved, including 100 males and 100 females, aged 38-78 years old, with an average age of 60 years old. There were 30 cases of breast cancer, 100 cases of digestive tract (esophagus, stomach, colorectal) cancers, 30 cases of lung cancer, 30 cases of urinary tract cancer, and 10 cases of multiple myeloma. Clinical stage: 70 cases in stage II, 40 cases in stage III, and 90 cases in stage IV.

Treatment method: The anti-tumor pharmaceutical products for combined immunization according to the present invention prepared in Example 1 was orally administered to the whole patient cases every day, in which a course of treatment was 120 days, and the specific dosage per day is shown in Table 8, wherein walnut extract capsule is expressed by I, wormwood extract capsule is expressed by II, and clove extract capsule is expressed by III.

Table 8 The dosing scheme for the first stage (20 days) (enhanced dose) of each course of treatment

| Day | I | | | II | | | III | | |
|---|---|---|---|---|---|---|---|---|---|
| | Morning | Noon | Evening | Morning | Noon | Evening | Morning | Noon | Evening |
| 1 | 1 | / | / | 1 | / | / | 1 | 1 | 1 |
| 2 | 1 | / | / | 1 | 1 | / | 2 | 2 | 2 |
| 3 | 1 | 1 | / | 1 | 1 | / | 3 | 3 | 3 |
| 4 | 1 | 1 | / | 1 | 1 | 1 | 3 | 3 | 3 |
| 5 | 1 | 1 | / | 1 | 1 | 1 | 3 | 3 | 3 |
| 6 | 1 | 1 | 1 | 1 | 1 | 1 | 3 | 3 | 3 |
| 7 | 1 | 1 | 1 | 1 | 1 | 2 | 3 | 3 | 3 |
| 8 | 1 | 1 | 2 | 1 | 1 | 2 | 3 | 3 | 3 |
| 9 | 1 | 1 | 2 | 1 | 2 | 2 | 3 | 3 | 3 |
| 10 | 1 | 2 | 2 | 2 | 2 | 2 | 1 | 1 | 1 |
| 11 | 1 | 2 | 2 | 2 | 2 | 2 | 1 | 1 | 1 |
| 12 | 2 | 2 | 2 | 2 | 2 | 3 | 1 | 1 | 1 |
| 13 | 2 | 2 | 2 | 2 | 2 | 3 | 1 | 1 | 1 |
| 14 | 2 | 2 | 3 | 2 | 2 | 3 | 1 | 1 | 1 |
| 15 | 2 | 2 | 3 | 2 | 2 | 3 | 1 | 1 | 1 |
| 16 | 2 | 3 | 3 | 2 | 2 | 3 | / | 1 | 1 |
| 17 | 2 | 3 | 3 | 2 | 2 | 3 | / | 1 | 1 |
| 18 | 2 | 3 | 3 | 2 | 2 | 3 | / | 1 | 1 |
| 19 | 3 | 3 | 3 | 2 | 2 | 2 | / | 1 | 1 |
| 20 | 3 | 3 | 3 | 2 | 2 | 2 | / | 1 | 1 |
| Total | 100 | | | 100 | | | 100 | | |

Table 9 The dosing scheme for the second stage (100 days) (maintenance dose) of each course of treatment

| Day | I | II | III | Day | I | II | III | Day | I | II | III | Day | I | II | III |
|---|---|---|---|---|---|---|---|---|---|---|---|---|---|---|---|
| 1 | 2 | / | 2 | 26 | 2 | 8 | 2 | 51 | 2 | / | 2 | 76 | 2 | 8 | 2 |
| 2 | 2 | 8 | 2 | 27 | 2 | / | 2 | 52 | 2 | 8 | 2 | 77 | 2 | / | 2 |
| 3 | 2 | / | 2 | 28 | 2 | / | 2 | 53 | 2 | / | 2 | 78 | 2 | / | 2 |
| 4 | 2 | / | 2 | 29 | 2 | / | 2 | 54 | 2 | / | 2 | 79 | 2 | / | 2 |
| 5 | 2 | / | 2 | 30 | 2 | 8 | 2 | 55 | 2 | / | 2 | 80 | 2 | 8 | 2 |
| 6 | 2 | 8 | 2 | 31 | 2 | / | 2 | 56 | 2 | 8 | 2 | 81 | 2 | / | 2 |
| 7 | 2 | / | 2 | 32 | 2 | / | 2 | 57 | 2 | / | 2 | 82 | 2 | / | 2 |
| 8 | 2 | / | 2 | 33 | 2 | / | 2 | 58 | 2 | / | 2 | 83 | 2 | / | 2 |
| 9 | 2 | / | 2 | 34 | 2 | 8 | 2 | 59 | 2 | / | 2 | 84 | 2 | 8 | 2 |
| 10 | 2 | 8 | 2 | 35 | 2 | / | 2 | 60 | 2 | 8 | 2 | 85 | 2 | / | 2 |
| 11 | 2 | / | 2 | 36 | 2 | / | 2 | 61 | 2 | / | 2 | 86 | 2 | / | 2 |

(continued)

| Day | I | II | III | Day | I | II | III | Day | I | II | III | Day | I | II | III |
|-----|---|----|----|-----|---|----|----|-----|---|----|----|-----|---|----|----|
| 12 | 2 | / | 2 | 37 | 2 | / | 2 | 62 | 2 | / | 2 | 87 | 2 | / | 2 |
| 13 | 2 | / | 2 | 38 | 2 | 8 | 2 | 63 | 2 | / | 2 | 88 | 2 | 8 | 2 |
| 14 | 2 | 8 | 2 | 39 | 2 | / | 2 | 64 | 2 | 8 | 2 | 89 | 2 | / | 2 |
| 15 | 2 | / | 2 | 40 | 2 | / | 2 | 65 | 2 | / | 2 | 90 | 2 | / | 2 |
| 16 | 2 | / | 2 | 41 | 2 | / | 2 | 66 | 2 | / | 2 | 91 | 2 | / | 2 |
| 17 | 2 | / | 2 | 42 | 2 | 8 | 2 | 67 | 2 | / | 2 | 92 | 2 | 8 | 2 |
| 18 | 2 | 8 | 2 | 43 | 2 | / | 2 | 68 | 2 | 8 | 2 | 93 | 2 | / | 2 |
| 19 | 2 | / | 2 | 44 | 2 | / | 2 | 69 | 2 | / | 2 | 94 | 2 | / | 2 |
| 20 | 2 | / | 2 | 45 | 2 | / | 2 | 70 | 2 | / | 2 | 95 | 2 | / | 2 |
| 21 | 2 | / | 2 | 46 | 2 | 8 | 2 | 71 | 2 | / | 2 | 96 | 2 | 8 | 2 |
| 22 | 2 | 8 | 2 | 47 | 2 | / | 2 | 72 | 2 | 8 | 2 | 97 | 2 | / | 2 |
| 23 | 2 | / | 2 | 48 | 2 | / | 2 | 73 | 2 | / | 2 | 98 | 2 | / | 2 |
| 24 | 2 | / | 2 | 49 | 2 | / | 2 | 74 | 2 | / | 2 | 99 | 2 | / | 2 |
| 25 | 2 | / | 2 | 50 | 2 | 4 | 2 | 75 | 2 | / | 2 | 100 | 2 | 4 | 2 |

*The maintenance dose was daily dose, and was not required to be distinguished as the morning, noon and evening doses. Also, the dosage of each type of capsule in the second stage was 200 capsules.

1. Changes in KPS score after treatment

According to the KPS scoring rules, the KPS scores of the cancer patients in this group were compared before and after the administration of the anti-tumor pharmaceutical products for combined immunization according to the present invention, and the comparison data is shown in Table 10.

Table 10 Effect on KPS scores in medium and advanced cancer patients

| | Changes in KPS score after treatment | | | | | |
|---|------|------|---|-----|-----|-----|
| | -20 | -10 | 0 | +10 | +20 | +30 |
| Number of patients | 0 | 0 | 10 | 150 | 40 | 0 |
| Ratio | 0 | 0 | 5% | 75% | 20% | 0 |

* "-" indicates a decrease in score compared to that before treatment;
"+" indicates an increase in score compared to that before treatment.

As can be seen from Table 10, after treatment with the anti-tumor pharmaceutical products for combined immunization, 95% of the patients scored higher, of which 20% were increased by 20 points or more.

2. Assessment of quality of life after treatment

The common symptoms of various types of tumors, such as general lassitude, poor appetite, vexation and insomnia, nausea, vomiting, etc., were mainly observed and the changes in the symptoms before and after treatment were compared.
According to the severity of symptoms (Table 11), after treatment, the score of ≥ 2 indicates a significant improvement, the score of 1 indicates a partial improvement, and no change in score indicates ineffectiveness. The specific results are shown in Table 12.

Table 11 Symptoms Scale

| Symptom | No symptom - | Level 1 + | Level 2 ++ | Level 3 +++ |
|---|---|---|---|---|
| Cough | No | Intermittent cough during the day, which does not affect normal life | Between level 1 and level 3 | Frequently coughs day and night or paroxysmal cough, which affects work and sleep |
| Bloody phlegm | No | There are blood streaks in sputum | There are blood clots in the sputum, accounting for 1/2, or bloody phlegm occurs less than 10 times every day | Bloody phlegm occurs above 10 times or hemoptysis |
| Shortness of breath | No | Shortness of breath occurs immediately after the activity, breathing difficulty (mild) | Breathing difficulty during rest (moderate) | Pant obviously and cannot lie down, which affects sleep and activity |
| Pain | No | Sudden onset, vague pain, which does not affect normal work | Frequent onset, heavier pain, which affects work | Repeated onset, severe pain, unbearable |
| Fever | No | 37.2-37.3°C | 37.6-38°C | More than 38.1°C |
| Expectoration | No | Expectoration occurs day and night, 10-60 mL | Expectoration occurs day and night, 60-100 mL | Expectoration occurs day and night, more than 100 mL |
| General lassitude | No | Slight general lassitude | Easy to fatigue, limb weakness | Limb weakness, lethargy |
| Loss of appetite | No | Unreduced appetite, but feels tasteless | Food intake reduced by 1/3 | Food intake reduced by 1/2 |
| Dry mouth and throat | No | Slightly dry mouth, less water intake | more obviously dry mouth, water intake is 1/2 to 1 times higher than usual water intake | obviously dry mouth, water intake is more than 1 time higher than usual water intake |
| Palpitation | No | Occasional palpitation | Frequent palpitation, more than three times a day | Serious palpitation, which needs drug therapy |
| Vexation and insomnia | No | Occasional nervousness and insomnia | Emotional lability occurs sometimes, irritability, restless sleep | irritability, prone to insomnia |
| Spontaneous sweat and night sweat | No | Occasionally spontaneous sweat and night sweat | Sweat after activity and night sweat | Spontaneous sweat and night sweat during the rest, which produces much sweat |
| Nausea and vomiting | No | Occasional nausea and vomiting | Frequent nausea, vomiting once or twice a day | Vomiting above 3 times a day |
| Tongue fur | Reddish | lightly red | Red, fat tongue, tooth mark around tongue | Red edge, tooth mark around tongue |
| Pulse | Normal | Small and wiry, soft | Wiry, thready rapid, soft and rolling | Small and weak, soft and small |

Table 12 Effect of the anti-tumor pharmaceutical products for combined immunization according to the present invention on quality of life scores in medium and advanced cancer patients

| | Effect on quality of life scores after treatment | | |
|---|---|---|---|
| | Significant improvement | Partial improvement | Ineffective |
| Number of patients | 140 | 60 | 0 |
| Ratio | 70% | 30% | 0 |

As can be seen from Table 12, after treatment with the anti-tumor pharmaceutical products for combined immunization, patients with a significant improvement in main symptoms reaches 70%.

3. Body weight change after treatment

After treatment, the patients whose weight gain or decrease ≥ 1 kg were considered as those having an increased or decreased weight, and the patients whose weight gain or decrease < 1 kg were considered as those having a stable weight. The results are shown in Table 13.

Table 13 Effect of the anti-tumor pharmaceutical products for combined immunization according to the present invention on body weight in medium and advanced cancer patients

| | Changes in body weight after treatment | | |
|---|---|---|---|
| | Decreased | Stable | Increased |
| Number of patients | 10 | 10 | 180 |
| Ratio | 5% | 5% | 90% |

As can be seen from Table 13, after treatment with the anti-tumor pharmaceutical products for combined immunization, 90% of patients gained weight, of which 55% increased by 2-3 kg.

4. Toxic and side reaction observation

No adverse reactions such as heart, liver and kidney function were observed in the whole patient cases during the treatment period, and no abnormalities were found in the peripheral blood. No allergic phenomena such as rash were observed.

The above description is only a preferred embodiment of the present invention, and the scope of protection of the present invention is not limited to the above embodiments. All the technical solutions under the inventive concept of the invention fall within the protection scope of the present invention. It should be noted that various modifications and refinements made by those skilled in the art without departing from the principles of the invention are also considered to be within the protection scope of the invention.

**Claims**

1. An anti-tumor pharmaceutical product for combined immunization, **characterized in that** the anti-tumor pharmaceutical product for combined immunization is prepared from an extract of a walnut, an extract of a wormwood, and an extract of a clove, the extract of a walnut, the extract of a wormwood, and the extract of a clove are in a weight ratio of 77:54:98.

2. The anti-tumor pharmaceutical product for combined immunization according to claim 1, **characterized in that** the walnut is black walnut, the wormwood is *Artemisia absinthium,* and the clove is clove bud.

3. The anti-tumor pharmaceutical product for combined immunization according to claim 1 or 2, **characterized in that** a formulation of the pharmaceutical product is a capsule.

4. The anti-tumor pharmaceutical product for combined immunization according to claim 1, **characterized in that** the anti-tumor pharmaceutical product for combined immunization further comprises paclitaxel.

5. A method of preparing the anti-tumor pharmaceutical product for combined immunization according to claim 3, **characterized in that** the method comprises preparation of a walnut extract capsule, preparation of a wormwood extract capsule, and preparation of a clove extract capsule; the preparation of a walnut extract capsule, the preparation of a wormwood extract capsule, and the preparation of a clove extract capsule include:

S1, screening and washing raw materials, and softening the clean raw materials by cold dipping under vacuum;
S2, subjecting the raw materials softened in the step S1 to supercritical fluid extraction and separating to obtain a liquid extract and a residue;
S3, pulverizing and grinding the residue obtained in step S2;
S4, spray drying the liquid extract obtained in step S2 and adding the pulverized and ground residue obtained in step S3 thereto during the spray drying to obtain a dried extract of raw materials; and
S5, filling a formulated amount of the extract of raw materials obtained in the step S4 into a capsule shell to obtain a capsule.

6. The method of preparing the anti-tumor pharmaceutical product for combined immunization according to claim 5, **characterized in that**:

in S1, the cold dipping under vacuum is performed at the temperature of 10-40°C and at the pressure of - 0.1 to -0.09 MPa;
in S2, a fluid for supercritical fluid extraction is carbon dioxide, and the extraction temperature is 30-40°C;
in S4, the temperature of spray drying is 40-80°C, the drying time is 3-60 s, and the extract of raw materials has a particle size of 10-60 μm; and
in S5, the capsule shell is a natural plant capsule shell.

7. A method of preparing the anti-tumor pharmaceutical product for combined immunization according to claim 3, **characterized in that**:

the method comprises preparation of a walnut extract capsule, preparation of a wormwood extract capsule, and preparation of a clove extract capsule;
the preparation of the walnut extract capsule comprises:

S1, screening and washing a walnut, and softening clean endodermis of the walnut by cold dipping under vacuum; the cold dipping under vacuum is performed at the temperature of 10-40°C and at the pressure of -0.1 to -0.09 MPa;
S2, subjecting the walnut softened in the step S1 to supercritical fluid extraction and separating to obtain a liquid extract of a walnut and a walnut residue; a fluid for supercritical fluid extraction is carbon dioxide, and the extraction temperature is 30-40°C;
S3, pulverizing and grinding the walnut residue obtained in step S2;
S4, spray drying the liquid extract of a walnut obtained in step S2 and adding the pulverized and ground walnut residue obtained in step S3 thereto during the spray drying to obtain a dried extract of a walnut; the temperature of spray drying is 40-80°C, the drying time is 3-60 s, and the extract of a walnut has a particle size of 10-60 μm; and
S5, filling 77 parts of the extract of a walnut obtained in the step S4 into a natural plant capsule shell to obtain the walnut extract capsule;

the preparation of the wormwood extract capsule comprises:

S1, screening and washing a wormwood, and softening clean whole herb of the wormwood by cold dipping under vacuum; the cold dipping under vacuum is performed at the temperature of 10-40°C and at the pressure of -0.1 to -0.09 MPa;
S2, subjecting the wormwood softened in the step S1 to supercritical fluid extraction and separating to obtain a liquid extract of a wormwood and a wormwood residue; a fluid for supercritical fluid extraction is carbon dioxide, and the extraction temperature is 30-40°C;
S3, pulverizing and grinding the wormwood residue obtained in step S2;
S4, spray drying the liquid extract of a wormwood obtained in step S2 and adding the pulverized and ground wormwood residue obtained in step S3 thereto during the spray drying to obtain a dried extract of a wormwood; the temperature of spray drying is 40-80°C, the drying time is 3-60 s, and the extract of a wormwood has a particle size of 10-60 μm; and

S5, filling 54 parts of the extract of a wormwood obtained in the step S4 into a natural plant capsule shell to obtain the wormwood extract capsule;

the preparation of the clove extract capsule comprises:

S1, screening and washing a clove, and softening clean and unopened flower buds of the clove by cold dipping under vacuum; the cold dipping under vacuum is performed at the temperature of 10-40°C and at the pressure of -0.1 to -0.09 MPa;

S2, subjecting the clove softened in the step S1 to supercritical fluid extraction and separating to obtain a liquid extract of a clove and a clove residue; the fluid for the supercritical fluid extraction is carbon dioxide, and the extraction temperature is 30-40°C;

S3, pulverizing and grinding the clove residue obtained in step S2;

S4, spray drying the liquid extract of the clove obtained in step S2 and adding the pulverized and ground clove residue obtained in step S3 thereto during the spray drying to obtain a dried extract of the clove; the temperature of spray drying is 40-80°C, the drying time is 3-60 s, and the extract of the clove has a particle size of 10-60 μm; and

S5, filling 98 parts of the extract of the clove obtained in the step S4 into a natural plant capsule shell to obtain the clove extract capsule; and

the walnut is black walnut, the wormwood is *Artemisia absinthium,* and the clove is clove bud.

8. Use of the anti-tumor pharmaceutical product for combined immunization according to claim 1 in the manufacture of a pharmaceutical product for preventing or treating cancers, **characterized in that** the cancers comprise intestinal cancer, mastocarcinoma, gastric cancer, liver cancer, prostate cancer, breast cancer, lung cancer, cervical cancer, ovarian cancer, kidney cancer, lymphoma, leukemia, skin cancer, melanoma, stromal cell tumor, myeloma or astrocytoma of the brain.

9. Use of the anti-tumor pharmaceutical product for combined immunization prepared by the method of preparing according to any of claims 5-7 in the manufacture of pharmaceutical products for preventing or treating cancers, **characterized in that** the cancers comprise intestinal cancer, mastocarcinoma, gastric cancer, liver cancer, prostate cancer, breast cancer, lung cancer, cervical cancer, ovarian cancer, kidney cancer, lymphoma, leukemia, skin cancer, melanoma, stromal cell tumor, myeloma or astrocytoma of the brain.

# INTERNATIONAL SEARCH REPORT

| International application No. |
|---|
| **PCT/CN2016/078651** |

### A. CLASSIFICATION OF SUBJECT MATTER

A61K 36/61 (2006.01) i; A61K 36/52 (2006.01) i; A61K 36/282 (2006.01) i; A61K 9/48 (2006.01) i; A61P 35/00 (2006.01) i
According to International Patent Classification (IPC) or to both national classification and IPC

### B. FIELDS SEARCHED

Minimum documentation searched (classification system followed by classification symbols)

A61K, A61P

Documentation searched other than minimum documentation to the extent that such documents are included in the fields searched

Electronic data base consulted during the international search (name of data base and, where practicable, search terms used)

WPI, EPODOC, CNPAT, CNKI: walnut, aromatic moxa, artemisiae argyi, artemisia, Artemisia absinthium, flos caryophyllata, Black Walnut, Juglans nigra L., Artemisia argyi Levi.et Vant., woormwood, Artemisia absinthium, Eugenia caryophyllata Thumb., paclitaxel, anti-tumor, combined immunization, simultaneous immunization, cancer, abstraction, extraction, supercritical fluid extraction, carbon dioxide, quench, dipping, atomized drying, drying spray

### C. DOCUMENTS CONSIDERED TO BE RELEVANT

| Category* | Citation of document, with indication, where appropriate, of the relevant passages | Relevant to claim No. |
|---|---|---|
| X | "Distinctive and Coordinated - Three Miraculous Anti-cancer Active Substances of WDS", CANCER REHABILITATION, no. 1, 28 February 2005 (28.02.2005), page 54, paragraph 1 and page 55, paragraphs 3-5 | 1-7 |
| Y | "An Ideal Choice of Cancer Rehabilitation Therapy – Pure Natural WDS Which Has Obtained Remarkable Achievements in the Past Eight-years Clinical Application (WDS)", CANCER REHABILITATION, no. 5, 31 October 2004 (31.10.2004), page 42, paragraph 3 | 8-9 |
| Y | "Distinctive and Coordinated - Three Miraculous Anti-cancer Active Substances of WDS" CANCER REHABILITATION, no. 1, 28 February 2005 (28.02.2005), page 54, paragraph 1 and page 55, paragraphs 3-5 | 8-9 |

☐ Further documents are listed in the continuation of Box C.     ☒ See patent family annex.

| | |
|---|---|
| *    Special categories of cited documents:<br><br>"A"  document defining the general state of the art which is not considered to be of particular relevance<br><br>"E"  earlier application or patent but published on or after the international filing date<br><br>"L"  document which may throw doubts on priority claim(s) or which is cited to establish the publication date of another citation or other special reason (as specified)<br><br>"O"  document referring to an oral disclosure, use, exhibition or other means<br><br>"P"  document published prior to the international filing date but later than the priority date claimed | "T"  later document published after the international filing date or priority date and not in conflict with the application but cited to understand the principle or theory underlying the invention<br><br>"X"  document of particular relevance; the claimed invention cannot be considered novel or cannot be considered to involve an inventive step when the document is taken alone<br><br>"Y"  document of particular relevance; the claimed invention cannot be considered to involve an inventive step when the document is combined with one or more other such documents, such combination being obvious to a person skilled in the art<br><br>"&"  document member of the same patent family |

| Date of the actual completion of the international search | Date of mailing of the international search report |
|---|---|
| 03 January 2017 (03.01.2017) | **10 January 2017 (10.01.2017)** |

| Name and mailing address of the ISA/CN:<br>State Intellectual Property Office of the P. R. China<br>No. 6, Xitucheng Road, Jimenqiao<br>Haidian District, Beijing 100088, China<br>Facsimile No.: (86-10) 62019451 | Authorized officer<br><br>**LU, Jianwei**<br><br>Telephone No.: (86-10) **62411990** |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1997361 B
- CN 103301201 B
- GB 478994 A
- GB 47891594 A